# EUROPEAN PATENT SPECIFICATION

(11) **EP 2 222 314 B1**
(45) Date of publication and mention of the grant of the patent: **09.01.2013**
(21) Application number: 08852743.7
(22) Date of filing: 18.11.2008
(51) Int. Cl.: A61K 33/18, A61K 33/44, A61P 11/06, A61P 11/08, A61P 11/00

(54) **USES AND MEANS FOR OTAINING BRONCHORELAXATION**
VERWENDUNG UND MITTEL ZUR BRONCHORELAXATION
USAGE ET MOYENS POUR OBTENIR UNE BRONCHORELAXATION

(30) Priority: 23.11.2007 SE 0702587
(43) Date of publication of application: 01.09.2010
(73) Proprietor: Pharmalundensis AB, 22184 Lund (SE)
(72) Inventor: SKOGVALL, Staffan, S-224 72 Lund (SE)
(74) Representative: Bergstrand, Mikael Gudmundsson
(86) International application number: PCT/SE2008/000644
(87) International publication number: WO 2009/067067

(56) References cited:
- EP-A1- 1 437 140
- WO-A1-96/04787
- WO-A1-99/60999
- WO-A1-2007/015102
- FR-A- 631 636
- GB-A- 2 255 718
- US-A- 1 879 601

## Description

### FIELD OF THE INVENTION

The present invention relates to a method and a means for obtaining bronchorelaxation in the airways of a human or an animal. The present invention also relates to a pharmaceutical composition and the use thereof for treating chronic obstructive pulmonary disease and asthma.

### BACKGROUND OF THE INVENTION

Chronic obstructive pulmonary disease (COPD) and asthma are important causes of morbidity, mortality and health-care costs worldwide. The estimated prevalence of COPD in many western countries is more than 10 % of the population (Mannino and Buist, 2007). In the USA, COPD was the primary reason for hospital discharge 9.8 million times and a secondary reason for discharge an additional 37.5 million times from 1979 to 2001. COPD is estimated to cause more than 80,000 deaths every year in the USA. It has been estimated that the total national cost in the USA for COPD was US$ 32.1 billion for the year 2003.

Approximately 300 million people worldwide currently have asthma. Most are found in the industrialized countries, which have an asthma prevalence of ~10 % in adults and almost 20 % in children. The rate of emergency hospital admissions during the early 2000 was 10/100,000 each year in adults and 100/100,000 in young children in the UK (Anderson et al., 2007). Asthma causes 1,200 deaths each year in the United Kingdom alone. The financial burden of patients with asthma in different western countries amounts to around US$ 300 billion every year.

COPD is associated with tobacco smoking and is characterized by inflammation in the airways and a gradual decline in lung function. Often, the patients experience cough, sputum production and wheezing, as well as repeated bouts of pneumonia, often several times per winter. The airway obstruction is usually irreversible, which means that it persists in spite of treatment with corticosteroids and beta2-agonists. As the disease progresses during many years, the airway obstruction can become very severe, leading to severe dyspnea during both exercise and rest and, eventually, lung failure. At this stage, lung function examinations with spirometry usually reveal a loss of lung capacity by 50 % or more. Other severe symptoms often appear at this time as well, such as weight loss, depression and cardiac disease. The mortality risk is high in these patients. The only established pharmaceutical treatment for these patients is anti-cholinergics, which only gives minor effects. Steroids and bronchodilators have minimal beneficial effects.

Asthma is characterized by chronic inflammation in the airways with reversible airway obstruction and bronchial hyper-reactivity. In contrast to COPD, asthma is usually treatable with steroids and bronchodilators. However, 10 % of asthmatics have severe symptoms in spite of maximum treatment. There is also an overlap between COPD and asthma, often rendering a firm diagnosis difficult to obtain (Chang & Mosenifar, 2007).

COPD in horses (also known as heaves, broken wind, alveolar emphysema and equine asthma) is characterized by inflammation in the airways. It can be caused by dusty or mouldy hay, dust and moulds in bedding, or pollens, dust and other irritants in the environment, but the cause is often unknown. The horses show symptoms like coughing, increased respiration, laboured breathing and yellow nasal discharge. The symptoms range in severity from mild, to so severe that the horse appears listless, has difficulty breathing and develops a muscular "heave line" along the horse's barrel from taking a double exhalation (The Columbia Encyclopedia, 2007). COPD in horses is often treated with β-agonists but the bronchorelaxing effect by these drugs is poor (Törneke, K. and Ingvast-Larson, C. 1999).

There have also been reports about obstructive pulmonary diseases, mainly asthma, in other animals such as cats and dogs. As in humans, these animals get an obstruction of the airways when the bronchi fill up with mucous and go into spasms (bronchoconstriction). It is far more common in cats than dogs, and particularly in Siamese and Himalayan cat breeds (AnimalHospitals-USA, 2007).

Clearly, there are many individuals with COPD and asthma who urgently need better treatments for their disease.

In US 6,063,363 is disclosed a method of treating upper respiratory tract infections with potassium salts, including potassium iodide and bromide. The potassium salt is introduced into the lungs and/or the nasal area and/or the oral cavity as a liquid solution, nasal spray, etc. However, the effect on the upper respiratory tract infections is said to be caused by the potassium cation saturation of the cells and tissues involved in upper respiratory tract infection. The anions of the administrated salts are of no importance in the treatment. Furthermore, US 6,063,363 discloses treatment of infection and not of bronchoconstriction.

In WO 00/36915 is disclosed a method of treating chronic obstructive airway disease by administering an osmotically active compound such as a salt, including potassium iodide and potassium bromide, sugar, sugar alcohol or organic osmolyte to the afflicted airway surface. The osmotically active compound is administered to the airways in order to increase the volume of the liquid on airway surfaces. No bronchorelaxing effect is reported.

In US 6,696,041 and US 6,171,611 are disclosed the use of iodine-containing nasal solutions for the treatment of nasal congestion caused by, i.e., common cold, flu or sinusitis. Both disclosed iodine-containing nasal solutions may also contain various salts including sodium iodide. US 6,171,611 further discloses a mouthwash solution comprising iodine and iodine salts, including potassium and sodium iodide.

US patents nos. 5,910,318 and 5,955,101 disclose starch-iodine pharmaceutical formulations for the preparation of capsules and tablets. The pharmaceutical formulations are suitable for administration to patients suffering from iodine deficiency diseases, in particular breast dysplasia, breast cancer, endometriosis, premenstrual syndrome, ovarian cysts and radiation sickness. In the formulation iodine is complexed with starch containing amylose, forming triiodide ions or polyiodide ions (I₅⁻up to I₁₁⁻). Iodine is released in the upper small bowel after hydrolysis of the starch by α-amylase. Since the triiodide ions cannot exist in non-complexed form I₂ is released.

Iodine toxicity may be a concern when high amounts of iodine are administered to a human. Iodine toxicity is manifested by, among other symptoms, thyroiditis, goiter, hypothyroidism and hyperthyroidism. It has been suggested that some individuals can tolerate very high levels of iodine with no apparent side effects and that iodine intakes less than or equal to 1,000 mg/day are probably safe for the majority of the population, but may cause adverse effects in some individuals (Pennington, 1990). Administration of a bromide salt, in particular sodium bromide, to animals suffering from the effects of iodine toxicity helps to reverse the symptoms (Baker et al., 2003). Sodium bromide is well tolerated by humans and it has been found to have a no-effect level of 4 mg/kg body weight (van Gelderen et al., 1993).

Activated charcoal is used in medical applications to treat poisoning and oral overdose of various medicaments. Activated charcoal has a very large surface area; 1 gram has a surface area of 300-2000 m² (Greenwood et al., 1984).
Impregnated activated charcoals are carbonaceous adsorbents which have chemicals finely distributed on their internal surface. The impregnation optimizes the existing properties of the activated charcoal giving a synergism between the chemicals and the charcoal (Carbo Tech-Aktivkohlen GmbH, Germany). Iodinated activated charcoal has been used for many years to bind heavy metals in gas.

### OBJECTS OF THE INVENTION

It is an object of the present invention to provide a means for producing bronchorelaxation in a human or an animal lung affected by airway obstruction.

Another object of the present invention is to provide bronchorelaxation in a human or an animal with chronic obstructive pulmonary disease.

A further object of the present invention is to provide bronchorelaxation in a human or an animal with asthma.

A still further object of the present invention is to provide a pharmaceutical composition for use in producing bronchorelaxation in a human or an animal lung affected by airway obstruction.

An additional object of the present invention is to provide a pharmaceutical composition for use in producing bronchorelaxation in humans or animals with chronic obstructive pulmonary disease.

Another object of the present invention is to provide a pharmaceutical composition for use in producing bronchorelaxation in humans or animals with asthma.

Further objects of the invention will become evident from the following summary of the invention, preferred embodiments and the appended claims.

### SUMMARY OF THE INVENTION

According to the present invention is disclosed the use of a pharmacologically effective amount of elemental iodine, I₂, on activated charcoal in the manufacture of a medicament for producing bronchorelaxation in the lungs of a human or an animal affected by airway obstruction.

In this specification the term iodine refers to elemental iodine, I₂, and the term iodine on activated charcoal refers to iodinated activated charcoal.

According to the present invention is also disclosed the use of a pharmacologically effective amount of elemental iodine, I₂, on activated charcoal in the manufacture of a medicament for producing bronchorelaxation in the lungs of a human or an animal with chronic obstructive pulmonary disease (COPD) and/or asthma.

According to the present invention is furthermore disclosed a pharmaceutical composition comprising iodine on activated charcoal for the above use.

Preferred administration forms for the pharmaceutical composition of the invention are tablets, tablets with disintegrants, capsules which disintegrate relatively fast in the stomach such as gelatin capsules and pullulan capsules, wherein the tablets and capsules comprise iodine on activated charcoal and optionally comprise a bromide salt and any of flavor, colour, preservative, sweetener excipient.

Preferred iodine concentration is from 1 % to 10 % w/w of the activated charcoal, in particular from 2 % to 8 % w/w of the activated charcoal, most preferred from 3 to 7 w/w of the activated charcoal.

Preferred daily doses to a human of iodine administered in form of the pharmaceutical composition of the invention are from 5 mg to 5,000 mg, in particular from 25 mg to 1,000 mg, most preferred from 50 mg to 250 mg.

Preferred daily doses of iodine administered to an animal in form of the pharmaceutical composition of the invention are from 0.07 mg/kg to 70 mg/kg body weight, in particular from 0.35 mg/kg to 15 mg/kg body weight, most preferred from 0.7 mg/kg to 3.5 mg/kg body weight.

Preferred doses administered to a human of activated charcoal are from 0.10 g to 100 g daily, in particular from 0.50 g to 20 g daily, most preferred from 1 g to 5 g daily.

Preferred daily doses administered to an animal of activated charcoal are from 1.5 mg/kg to 1,400 mg/kg body weight, in particular from 7 mg/kg to 285 mg/kg body weight, most preferred from 14 mg/kg to 70 mg/kg body weight.

According to a first preferred aspect of the invention iodinated activated charcoal is administered to the intestine of a human or an animal in need of bronchorelaxation, in a pharmaceutically acceptable form, in particular in form of a tablet or capsule comprising elemental iodine on activated charcoal.

According to a second preferred aspect of the invention the iodinated activated charcoal is administered to the intestine of a human or an animal in form of a tablet, wherein the tablet comprises disintegrant for fast release of the tablet contents in the stomach.

According to a third preferred aspect of the invention the iodinated activated charcoal is administered to the intestine of a human or an animal in form of a capsule, wherein the capsule shell is comprised by gelatin or pullulan for fast release of the capsule contents.

According to a fourth preferred aspect of the invention a bromide salt is co-administered to a human or an animal in need of bronchorelaxation with the iodinated activated charcoal to minimize the risk of iodine toxicity.

Preferred bromide salts are sodium, potassium, magnesium, lithium, ammonium and calcium bromide.

A preferred concentration of the co-administered bromide salt is from 0.5 % to 5 % w/w of the iodinated activated charcoal.

The invention will now be described in more detail by reference to preferred but not limiting embodiments.

### DESCRIPTION OF PREFERRED EMBODIMENTS OF THE INVENTION

### EXAMPLE 1. Administration of iodine on activated charcoal.

A male Caucasian, born 1935, had been smoking cigarettes daily for many years, but quit about 10 years ago. The decision to quit smoking was caused by increasing problems from the airways, with repeated episodes of pneumonia and airway obstruction. These symptoms were usually treated with antibiotics, steroids and bronchodilators. The COPD diagnose was first suggested in June 2000.

After that, the airway symptoms increased considerably, with month-long episodes of cough and exercise-induced dyspnea. Spirometric evaluation some years later showed a Forced Expiratory Volume in one second, FEV₁, of 1.44 L, corresponding to 49.7 % of his reference value, and a Peak Expiratory Flow, PEF, of 282 L/min, corresponding to 60.1 % of his reference value.

In the following months the situation continued to worsen with loss of appetite, reduction of weight and severe dyspnea during rest, in spite of maximum treatment with anti-cholinergics, steroids and bronchodilators. The patient now felt desperately ill, and questioned how long he would be able to survive.

The severity of his condition prompted the patient to look for alternative treatments. When ingesting iodinated activated charcoal, in form of rods (1×1×5 mm) of activated charcoal comprising about 5 % iodine by weight, I₂, (Sigma-Aldrich, Inc.) the patient experienced immediate relief of airway obstruction and dyspnea. A few days intake of 1-2 g iodinated activated charcoal twice per day (equivalent of 50-100 mg iodine twice per day), suspended in yoghurt, produced a dramatic increase in lung function and stamina, and completely removed the dyspnea. Sputum production was also considerably reduced. A renewed spirometric evaluation a few months later confirmed the subjective improvements (FEV₁ = 2.79 L, corresponding to 97.1 % of his reference value and PEF = 427 L/min, corresponding to 92.5 % of his reference value).

The patient has continued to regularly take the combination for more than a year and still experiences the full benefit of it. A temporary discontinuation of the intake for a few days led to the reappearance of many of the symptoms. However, they quickly disappeared upon the resumption of the intake of iodinated activated charcoal. The patient now lives a normal life, being able to pursue gardening, cycling and even to play an occasional game of badminton.

To test the bronchorelaxing effect of activated charcoal without addition of iodine, the same patient took 5 g of iodine-free activated charcoal (Medikol, Selena Fournier) for a few days. However, no bronchorelaxing effect was observed, instead his condition worsened.

To clarify if iodine in itself has bronchorelaxing properties, 50 mg elemental iodine (Sigma-Aldrich, Inc.), placed in a gelatin capsule, was taken by the same patient for a few days. However, no distinct bronchorelaxing effect was observed.

### EXAMPLE 2. Tablets comprising iodine on activated charcoal.

Tablets comprising iodine on activated charcoal were compressed in a conventional tabletting machine from 500 mg iodinated activated charcoal (Sigma-Aldrich, Inc.) mixed with 122 mg lactose monohydrate, 6 mg magnesium stearate and 122 mg sodium methyl cellulose to form a 750 mg tablet comprising about 25 mg iodine. Optionally, sodium bromide (0.5-5 % w/w) can be added to the tabletting mixture.

### EXAMPLE 3. Tablets comprising iodine on activated charcoal - for fast release in the stomach.

Tablets comprising iodine on activated charcoal for fast release of the tablet content in the stomach were compressed in a conventional tabletting machine from 500 mg iodinated activated charcoal (Sigma-Aldrich, Inc.) mixed with 108 mg lactose monohydrate, 6 mg magnesium stearate, 16 mg croscarmellose sodium and 120 mg sodium methyl cellulose to form a 750 mg tablet comprising about 25 mg iodine. Optionally, sodium bromide (0.5-5 % w/w) can be added to the tabletting mixture.

### EXAMPLE 4. Capsules comprising iodine on activated charcoal and sodium bromide.

Capsules comprising iodine on activated charcoal and sodium bromide were manufactured by mixing 350 mg iodinated activated charcoal (Sigma-Aldrich, Inc.) with 5 mg sodium bromide. Gelatin capsules were filled with the mixture in a conventional capsule filling machine to form capsules containing about 17 mg iodine.

### EXAMPLE 5. Capsules comprising iodine on activated charcoal and sodium bromide - for fast release in the stomach.

Capsules comprising iodine on activated charcoal and sodium bromide were manufactured by mixing 350 mg iodinated activated charcoal (Sigma-Aldrich, Inc.) with 5 mg sodium bromide. Pullulan capsules were filled with the mixture in a conventional capsule filling machine to form capsules containing about 17 mg iodine.

### EXAMPLE 6. Absorption by activated charcoal of Hg dissolved in water.

A solution of metallic Hg in water was prepared by placing a droplet of mercury in a 250 ml beaker, adding 150 ml of distilled water, and stirring for 1 h at 40 °C. 100 mL of the aqueous phase was decanted into another 250 ml beaker and a sample of 30 mL was withdrawn. Iodinated activated charcoal (0.7 g) was added to the aqueous phase and the suspension stirred at 40°C; samples (30 ml each) were taken at 30 min and 60 min. The samples were filtered into glass tubes provided with polypropylene stoppers, 2 % nitric acid (0.6 ml) was added to each sample, and the samples sent for analysis. Hg²⁺, mg/mL: 0.075 prior to addition of iodine on charcoal; 0.0055 after 30 min; < 0.0001 after 60 min. Thus, 1 h exposure to 1 g iodinated activated charcoal/100 ml water removed 99.9 % of Hg (0) from the solution.

### References

Mannino, D. M. and Buist, S. A. Gobal burden of COPD: risk factors, prevalence, and future trends. Lancet 2007 370:765-73.
Anderson, R. H., Ramyani, G., Strachan, D. P., and Limb, E. S. 50 years of Asthma: UK trends from 1955 to 2004. Thorax 2007 62:85-90.
Chang, J. and Mosenifar, Z. Differentiating COPD from Asthma in Clinical Practice. Journal of Intensive Care Medicine 2007 22:300-309.
Törneke, K. and Ingvast-Larson, C. Beta2-agonister vid behandling av COPD på häst. Svensk Veterinärtidning 1999 51(1):13-16.
The Columbia Encyclopedia, 6th Ed., Columbia University Press, USA, http://www.encyclopedia.com/doc/1E1-heaves.html, accessed Nov. 19, 2007.
AnimalHospitals-USA, http://www.animalhospitals-usa.com/dogs/asthma.html, accessed Nov. 19, 2007.
Pennington, J. A. A Review of Iodine Toxicity Reports. Journal of the American Dietetic Association 1990 90(11):1571-81.
Baker, D. H., Parr, T. H. and Augspurger, N. R. Oral Iodine Toxicity in Chicks Can Be Reversed by Supplemental Bromine. Journal of Nutrition 2003 133:2309-2312.
van Gelderen, C. E., Savelkoul, T. J., Blom, J. L., van Dokkum, W. and Kroes, R. The No-effect Level of Sodium Bromide in Healthy Volunteers. Human & Experimental Toxicology 1993 12(1):9-14.
Greenwood, N. N. and Earnshaw, A. Chemistry of the Elements. Pergamon Press, 1984*.*
Carbo Tech-Aktivkohlen GmbH, Franz-Ficher-Weg 61, D-45307 Germany.

## Claims

1. A pharmaceutical composition for use for producing bronchorelaxation in a human or an animal lung affected by airway obstruction, comprising a pharmacologically effective amount of elemental iodine on activated charcoal (iodinated activated charcoal).

2. The pharmaceutical composition for use of claim 1, wherein the airway obstruction is caused by chronic obstructive pulmonary disease.

3. The pharmaceutical composition for use of claim 1, wherein the airway obstruction is caused by asthma.

4. The pharmaceutical composition for use of claim 1, comprising a bromide salt for coadministration with the elemental iodine on activated charcoal.

5. The pharmaceutical composition for use of claim 1, wherein the bromide salt is sodium bromide, potassium bromide, magnesium bromide, lithium bromide, ammonium bromide or calcium bromide.

6. The pharmaceutical composition for use of claim 1, wherein the bromide salt is in an amount of from 0.5 to 5 % w/w of the elemental iodine on activated charcoal.

7. The use of elemental iodine on activated charcoal (iodinated activated charcoal) for the manufacture of a medicament for producing bronchorelaxation in a human or an animal airways affected by obstruction.

8. The use of elemental iodine on activated charcoal (iodinated activated charcoal) for the manufacture of a medicament for producing bronchorelaxation in a human or an animal with chronic obstructive pulmonary disease.

9. The use of elemental iodine on activated charcoal (iodinated activated charcoal) for the manufacture of a medicament for producing bronchorelaxation in a human or an animal with asthma.

10. The use of any of claims 7 to 9, wherein the medicament comprises a bromide salt.

11. The use of claim 10, wherein the bromide salt is sodium bromide, potassium bromide, magnesium bromide, lithium bromide, ammonium bromide or calcium bromide.

12. The use of any of claims 10 to 11, wherein the bromide salt is in an amount of from 0.5 to 5 w/w of the elemental iodine on activated charcoal.

## Patentansprüche

1. Pharmazeutische Zusammensetzung zur Verwendung bei der Herstellung der Bronchorelaxation in einer menschlichen oder einer tierischen Lunge, die von einer Atemwegsobstruktion betroffen ist, umfassend eine pharmakologisch wirksame Menge elementaren lods auf Aktivkohle (Iodierte Aktivkohle).

2. Pharmazeutische Zusammensetzung zur Verwendung nach Anspruch 1, wobei die Atemwegsobstruktion durch chronisch-obstruktive Lungenerkrankung verursacht wird.

3. Pharmazeutische Zusammensetzung zur Verwendung nach Anspruch 1, wobei wobei die Atemwegsobstruktion durch Asthma verursacht wird.

4. Pharmazeutische Zusammensetzung zur Verwendung nach Anspruch 1, umfassend ein Bromidsalz zur gleichzeitigen Gabe mit dem elementaren Iod auf Aktivkohle.

5. Pharmazeutische Zusammensetzung zur Verwendung nach Anspruch 1, wobei es sich bei dem Bromidsalz um Natriumbromid, Kaliumbromid, Magnesiumbromid, Lithiumbromid, Ammoniumbromid oder Calciumbromid handelt.

6. Pharmazeutische Zusammensetzung zur Verwendung nach Anspruch 1, wobei das Bromidsalz in einer Menge von 0,5 bis 5 % w/w des elementaren Iods auf Aktivkohle vorliegt.

7. Verwendung von elementarem Iod auf Aktivkohle (iodierter Aktivkohle) für die Herstellung eines Medikaments zur Herstellung der Bronchorelaxation in menschlichen oder tierischen Atemwegen, die von einer Obstruktion betroffen sind.

8. Verwendung von elementarem Iod auf Aktivkohle (iodierter Aktivkohle) für die Herstellung eines Medikaments zur Herstellung der Bronchorelaxation bei einem Menschen oder einem Tier, welcher/welches von einer chronisch-obstruktiven Lungenerkrankung betroffen sind.

9. Verwendung von elementarem Iod auf Aktivkohle (iodierter Aktivkohle) für die Herstellung eines Medikaments zur Herstellung der Bronchorelaxation bei einem Menschen oder einem Tier mit Asthma.

10. Verwendung nach Anspruch 7 bis 9, wobei das Medikament ein Bromidsalz umfasst.

11. Verwendung nach Anspruch 10, wobei es sich bei dem Bromidsalz um Natriumbromid, Kaliumbromid, Magnesiumbromid, Lithiumbromid, Ammoniumbromid oder Calciumbromid handelt.

12. Verwendung nach einem der Ansprüche 10 bis 11, wobei das Bromidsalz in einer Menge von 0,5 bis 5 % w/w des elementaren Iods auf Aktivkohle vorliegt.

## Revendications

1. Composition pharmaceutique à utiliser pour produire une bronchorelaxation dans un poumon humain ou animal affecté par une obstruction des voies aériennes, comprenant une quantité pharmacologiquement efficace d'iode élémentaire sur charbon activé (charbon activé iodé).

2. Composition pharmaceutique à utiliser selon la revendication 1, dans laquelle l'obstruction des voies aériennes est provoquée par une maladie pulmonaire obstructive chronique.

3. Composition pharmaceutique à utiliser selon la revendication 1, dans laquelle l'obstruction des voies aériennes est provoquée par de l'asthme.

4. Composition pharmaceutique à utiliser selon la revendication 1, comprenant un sel de bromure pour une coadministration avec l'iode élémentaire sur charbon activé.

5. Composition pharmaceutique à utiliser selon la revendication 1, dans laquelle le sel de bromure est du bromure de sodium, du bromure de potassium, du bromure de magnésium, du bromure de lithium, du bromure d'ammonium ou du bromure de calcium.

6. Composition pharmaceutique à utiliser selon la revendication 1, dans laquelle le sel de bromure est dans une quantité allant de 0,5 à 5 % poids/poids de l'iode élémentaire sur charbon activé.

7. Utilisation d'iode élémentaire sur charbon activé (charbon activé iodé) pour la fabrication d'un médicament pour produire une bronchorelaxation dans des voies aériennes humaines ou animales affectées par une obstruction.

8. Utilisation d'iode élémentaire sur charbon activé (charbon activé iodé) pour la fabrication d'un médicament pour produire une bronchorelaxation chez un humain ou un animal atteint d'une maladie pulmonaire obstructive chronique.

9. Utilisation d'iode élémentaire sur charbon activé (charbon activé iodé) pour la fabrication d'un médicament pour produire une bronchorelaxation chez un humain ou un animal atteint d'asthme.

10. Utilisation selon l'une quelconque des revendications 7 à 9, dans laquelle le médicament comprend un sel de bromure.

11. Utilisation selon la revendication 10, dans laquelle le sel de bromure est du bromure de sodium, du bromure de potassium, du bromure de magnésium, du bromure de lithium, du bromure d'ammonium ou du bromure de calcium.

12. Utilisation selon l'une quelconque des revendications 10 à 11, dans laquelle le sel de bromure est dans une quantité allant de 0,5 à 5 poids/poids d'iode élémentaire sur charbon activé.
